Europäisches Patentamt

European Patent Office

Office européen des brevets

(19)

(11) Veröffentlichungsnummer: **0 260 527 B1**

## (12) EUROPÄISCHE PATENTSCHRIFT

(45) Veröffentlichungstag der Patentschrift: **01.04.92**

(21) Anmeldenummer: **87112773.4**

(22) Anmeldetag: **02.09.87**

(51) Int. Cl.5: **A61K 45/00**, A61K 31/505,
A61K 31/47, A61K 31/415,
A61K 31/535, //(A61K31/505,
31:19,31:18),(A61K31/47,31:19,
31:18),(A61K31/415,31:19,
31:18),(A61K31/535,31:19,
31:18)

(54) **Neue synergistische Kombination bestehend aus einem Phosphodiesterase-Hemmer und einem Tromboxan-A2-Antagonisten und deren Verwendung bzw. Herstellung.**

(30) Priorität: **13.09.86 DE 3631294**

(43) Veröffentlichungstag der Anmeldung:
**23.03.88 Patentblatt 88/12**

(45) Bekanntmachung des Hinweises auf die
Patenterteilung:
**01.04.92 Patentblatt 92/14**

(84) Benannte Vertragsstaaten:
**BE CH DE ES FR GB GR IT LI LU NL SE**

(56) Entgegenhaltungen:
**US-A- 4 568 676**

(73) Patentinhaber: **Dr. Karl Thomae GmbH
Postfach 1755
W-7950 Biberach (Riss)(DE)**

(72) Erfinder: **Haarmann, Walter, Dr.
Schlierholzweg 27
W-7950 Biberach 1(DE)**
Erfinder: **Nickl, Josef, Dr., Dipl.-Chem.
Silcherstrasse 8
W-7950 Biberach 1(DE)**

DIALOG 04913956 Medline 83146956; J.B. SMITH: "Effect of thromboxane sythetase inhibitors on platelet function: enhancement by inhibition of phosphodiesterase", & THROMB RES November 15 1982. 28(4) p477-85

DIALOG 06279900 Medline 87253900; E.F. SMITH et al.: "Comparison of the effects of a thromboxane synthase inhibitor or prostacyclin in combination with a phosphodiesterase inhibitor for the prevention of experimental thrombosis and sudden death in rabbits", & J PHARMACOL EXP. THER., Juni 1987. 241 (3) p855-60

CHEMICAL ABSTRACTS, Band 105, 15. September 1986, Seiten 39,40, Zusammenfassung Nr. 90990m, Columbus, Ohio, US; T. SILLS et al.: "Effects of a thromboxane synthetase inhibitor and a cAMP phosphodiesterase inhibitor, singly and in combination, on platelet behavior", & TROMB. HAEMOSTASIS 1986, 55(3), 305-8

J. CLIN. INVEST. 75 1591-1599 (1985) (D doc.)

**Beschreibung**

In der Literatur (siehe US-A-4,568,676, Thromb. Res. 28, 477-485 (1982) und Thromb. and Haemostasis 55, 305-308 (1986)) wird festgestellt, daß die Arzneimittelkombination, die einen Phosphodiesterasehemmer und einen Thromboxansynthetasehemmer enthält, eine synergistisch hemmende Wirkung auf die Thrombozytenaggregation in vitro aufweist.

Demgegenüber ist aus J. Clin. Invest. 75, 1591-1599 (1985) bekannt, daß die Kombination des Phosphodiesterase (PDE)-Hemmers Dipyridamol mit dem Thromboxan-Synthese-Hemmer Dazoxiben (4-[2-(1H-Imidazol-1-yl)-ethoxy]benzoesäure) keinen verbessernden Einfluß auf die antithrombotische Wirkung des PDE-Hemmers aufweist. Dieser Weg, die Behandlung und Prophylaxe von Thrombosen zu verbessern, war offensichtlich nicht erfolgreich, da die Rezeptoren für Thromboxan-A$_2$ nicht spezifisch sind. Diese reagieren auch mit Prostaglandin-H$_2$, dem Vorläufer von Thromboxan-A$_2$, dessen Synthese aber durch einen Thromboxan-A$_2$-Synthesehemmer nicht gehemmt wird.

Überraschenderweise wurde nun gefunden, daß die Kombination bestehend aus einem Phosphodiesterase-Hemmer und einem Thromboxan-A$_2$-Antagonisten eine viel stärkere antithrombotische Wirkung als die Einzelsubstanzen aufweist.

Gegenstand der vorliegenden Erfindung ist somit die neue synergistische Kombination bestehend aus einem PDE-Hemmer und einem Thromboxan A$_2$-Antagonisten und deren Verwendung sowie ein Verfahren zu deren Herstellung wie in den Ansprüchen im einzelnen gekennzeichnet.

In der Literatur werden zahlreiche PDE-Hemmer und Thromboxan A$_2$-Antagonisten beschrieben.

Als PDE-Hemmer seien daher beispielsweise die aus der Pyrimido-[5,4-d]pyrimidin-Reihe (siehe US-A-3.031.450, US-A-3.322.755 und US-A-4.518.596), aus der Isochinolinreihe (siehe US-A-3.975.524), aus der Pyrido[3,2-d]pyridiminreihe (siehe US-A-3.843.638), aus der Indolin- und Carbostyrilreihe (siehe US-A-4.329.347, GB-B-2.098.595 und US-A-4.442.111) und der Benzoxazin-2-on-Reihe (siehe US-A-4.518.597) erwähnt.

Als Einzelverbindungen seien hier folgende beispielsweise genannt:
Papaverin,
2,6-Bis(diäthanolamino)-4,8-dipiperidino-pyrimido[5,4-d]pyrimidin (Dipyridamol),
2,6-Bis(diäthanolamino)-4-piperidino-pyrimido[5,4-d]pyrimidin (Mopidamol),
6,7-Dichlor-1,5-dihydro-imidazo[2,1-b]chinazolin-2(3H)-on (Anagrelid),
7-Brom-1,5-dihydro-3,6-dimethyl-imidazo[2,1-b]chinazolin2(3H)-on,
6-[4-(1-Cyclohexyl-1H-5-tetrazolyl)-butoxy]-2-oxo-tetrahydrochinolin,
7-Äthoxy-carbonyl-6,8-dimethyl-4-hydroxymethyl-1(2H)-phthalazinon (Phthalazinol) und
3,4-Dihydro-1-methyl-6-(1,4,5,6-tetrahydro-4-methyl-6-oxo-3-pyridazinyl)-2(1H)-chinolinon.

Die perorale Tagesdosis beträgt beispielsweise
für Papaverin 1 bis 2 mg/kg, vorzugsweise 1,5 mg/kg,
für Dipyridamol 2,5 bis 7,5 mg/kg, vorzugsweise 5 mg/kg,
für Mopidamol 15 bis 25 mg/kg, vorzugsweise 20 mg/kg,
für Anagrelid 0,05 bis 0,15 mg/kg, vorzugsweise 0,1 mg/kg,
für 7-Brom-1,5-dihydro-3,6-dimethyl-imidazo[2,1-b]chinazolin-2(3H)-on 0,075 bis 0,15 mg/kg, vorzugsweise 0,1 mg/kg,
für 6-[4-(1-Cyclohexyl-1H-5-tetrazolyl)-butoxy]-2-oxo-tetrahydrochinolin 2,0 bis 4,0 mg/kg, vorzugsweise 3 mg/kg,
für Phthalazinol 2,5 bis 7,0 mg/kg, vorzugsweise 4,0 mg/kg und
für 3,4-Dihydro-1-methyl-6-(1,4,5,6-tetrahydro-4-methyl-6-oxo-3-pyridazinyl)-2(1H)-chinolinon 0,05 bis 0,15 mg/kg, vorzugsweise 0,1 mg/kg.

Als besonders geeignete PDE-Hemmer haben sich hierbei erfindungsgemäß die PDE-Hemmer aus der Pyrimido[5,4-d]pyrimidin-Reihe wie Dipyridamol oder Mopidamol erwiesen.

Als Thromboxan A$_2$-Antagonisten seien beispielsweise die aus der Benzolsulfonamidoäthyl-Reihe (siehe DE-A-2.809.377, DE-A-3.000.377 und EP-A-0.194.548) und aus der Heptensäurereihe (siehe NL-A-81/02.116, EP-A-0.044.711, C.A. 98, 638n und 192.381t (1983), C.A. 99, 1014p, (1983)) genannt.

Als Einzelverbindungen seien hierbei beispielsweise folgende genannt:
4-[2-(Benzolsulfonamido)-äthyl]-phenoxy-essigsäure,
3-[4-(2-Benzolsulfonylaminoäthyl)-benzoyl]propionsäure-Kaliumsalz,
3-[4-(2-(4-Fluorbenzolsulfonylamino)-äthyl)-benzoyl]-propionsäure-methylester,
3-[4-(2-(4-Fluorbenzolsulfonylamino)-äthyl)-benzoyl]-propionsäure,
3-[4-(2-(4-Chlorbenzolsulfonylamino)-äthyl)-benzoyl]-propionsäure-methylester,
3-[4-(2-(4-Chlorbenzolsulfonylamino)-äthyl)-benzoyl]-propionsäure,

3-[4-(2-(p-Toluolsulfonylamino)-äthyl)-benzoyl]propionsäuremethylester,

3-[4-(2-(p-Toluolsulfonylamino)-äthyl)-benzoyl]propionsäure,

3-[4-(2-(3-Pyridinsulfonylamino)-äthyl)-benzoyl]propionsäuremethylester,

3-[4-(2-(3-Pyridinsulfonylamino)-äthyl)-benzoyl]propionsäure,

3-[4-(2-(2-Thiophensulfonylamino)-äthyl)-benzoyl]-propionsäure-methylester,

3-[4-(2-(2-Thiophensulfonylamino)-äthyl)-benzoyl]-propionsäure,

3-[4-(2-(2′-Fluor-4-biphenylyl-sulfonylamino)-äthyl)-benzoyl]-propionsäure,

4-[4-(2-Benzolsulfonylaminoäthyl)-phenyl]-4-hydroxy-buttersäure,

3-[4-(2-Benzolsulfonylaminoäthyl)-benzoyl]acrylsäure,

3-[4-(2-Benzolsulfonylaminoäthyl)-benzoyl]buttersäure-äthylester,

3-[4-(2-Benzolsulfonylaminoäthyl)-benzoyl]buttersäure,

3-[4-(2-(p-Chlorbenzolsulfonylamino)-äthyl)-benzoyl]-buttersäure,

3-[4-(2-(p-Toluolsulfonylamino)-äthyl)-benzoyl]buttersäureäthylester,

3-[4-(2-(p-Toluolsulfonylamino)-äthyl)-benzoyl]buttersäure,

3-[4-(2-(3-Pyridylsulfonylamino)-äthyl)-benzoyl]buttersäure,

5-[4-(2-Benzolsulfonylaminoäthyl)-benzoyl]pentansäure-äthylester,

5-[4-(2-Benzolsulfonylaminoäthyl)-benzoyl]pentansäure,

3-[4-(2-(1-Naphthalinsulfonylamino)-äthyl)-benzoyl]-propionsäure-methylester,

3-[4-(2-(1-Naphthalinsulfonylamino)-äthyl)-benzoyl]-propionsäure,

3-[4-(2-Benzolsulfonylaminoäthyl)-naphthoyl-(1)]propionsäure,

3-[4-(2-Benzolsulfonylaminoäthyl)-benzoyl]propionsäure-äthylester,

3-[4-(2-Benzolsulfonylaminoäthyl)-benzoyl]propionsäure-methylester,

3-[4-(2-Benzolsulfonylaminoäthyl)-benzoyl]propionsäure-ß-methoxyäthylester,

[4-(2-Benzolsulfonylaminoäthyl)-benzoyl]essigsäureäthylester und

4-Hydroxy-4-[4-(2-benzolsulfonylaminoäthyl)-phenyl]crotonsäure.

Als besonders geeingete Thromboxan $A_2$-Antagonisten haben sich erfindungsgemäß die Thromboxan $A_2$-Antagonisten aus der Benzolsulfonamidoreihe wie

4-[2-(Benzolsulfonamido)-äthyl]-phenoxy-essigsäure und

3-[4-(2-(p-Toluolsulfonamido)-äthyl)-benzoyl]-propionsäure erwiesen.

Beispielsweise beträgt die perorale Tagesdosis für die vorstehend genannten Verbindungen 5,0 bis 20 mg/kg, vorzugsweise 7,5 bis 12 mg/kg.

Die synergistische Wirkung der neuen Kombination wurde beispielsweise an der Kombination Dipyridamol (Substanz A) 3-[4-(2-(p-Toluolsulfonamido)-äthyl)-benzoyl]propionsäure-Natriumsalz (Substanz B) im Vergleich zu den Einzelsubstanzen als eine die Thrombusbildung hemmende Wirkung an der Ratte wie folgt geprüft (siehe Poliwoda et al. in Z. Ges. exp. Med. 145, 252 (1968)):

Männliche Ratten im Gewicht von 70 - 90 g, die bis zum Versuchsbeginn freien Zugang zu Futter (Altromin ® ) und Wasser hatten, erhielten eine intraperitoneale Nembutal-Narkose (50 bis 60 mg/kg Pentobarbital-Natrium). Zur Vermeidung einer Hypothermie wurden sie auf einem geheizten Versuchstisch (37°C) gelagert.

Nach Anlegen eines transversalen Bauchschnittes wurde eine Dünndarmschlinge vorgelagert, fixiert und eine Mesenterialvene mit einem Durchmesser von 300 $\mu$m unter einem binokularen Operationsmikroskop mit 40facher Vergrößerung eingestellt. An die Vene wurde eine in Glas eingebettete monpolare V4A-Stahl-Elektrode von l00 $\mu$g Spitzendurchmesser als Reizkathode angelegt. Die Anode lag am gleichen Gefäß der Kathode gegenüber. Die Reizung geschah mit 150 Volt Gleichstrom für die Dauer von 100 msec. Nach der Reizung wurde das Beobachtungsgebiet kontinulierlich mit warmer (37°C) physiologischer Kochsalzlösung bespült.

Entstehung und Wachstum des Thrombus wurden unter dem Mikroskop über einen Zeitraum von 20 Minuten verfolgt. Während der gesamten Beobachtungsdauer wurde anfangs in kürzeren Abständen, später jede Minute die prozentuale Einengung des Gefäßdurchmessers durch den Thrombus ermittelt und protokolliert.

Die Werte wurden gegen die Zeit in ein Koordinatensystem eingetragen. Die Fläche unter der so erhaltenen Kurve ("area under curve")stellt ein Maß für die Thrombusgröße über die Zeit dar.

Gruppen von je 5 Tieren erhielten per os entweder

10 mg/kg Substanz A oder

2,5 mg/kg Substanz B oder

10 mg/kg Substanz A + 2,5 mg/kg Substanz B oder

das Vehikel.

Die Versuchsanordnung umfaßte also 4 Tiergruppen.

Die "area under curve" der substanzbehandelten Tiere wurde mit der der Kontrolltiere verglichen. Die Reduktion der Thrombusgröße unter Substanzeinfluß wurde erfaßt als prozentuale Verminderung des Mittelwertes der "area under curve" der substanzbehandelten Tiere über die gesamte Beobachtungszeit gegenüber dem Mittelwert der Kontrolle.

Die Signifikanz der Verminderung wurde berechnet nach dem t-Test für unabhängige Stichproben.

Die nachfolgende Tabelle enthält die gefundenen Ergebnisse:

| Substanz | n | Hemmwirkung |
|----------|---|-------------|
| A | 5 | - 11 % |
| B | 5 | - 31 % |
| A + B | 5 | - 66 % |

Die an der Ratte geprüfte Kombination, nämlich 10 mg/kg Substanz A + 2,5 mg/kg Substanz B, ist nicht toxisch, da bei ihrer Applikation keine toxischen Nebenwirkungen beobachtet werden konnten.

Auf Grund ihrer pharmakologischen Eigenschaften eignet sich die neue Kombination zur Behandlung und Prophylaxe von venösen und arteriellen Thrombosen, also zur Verhütung oder Behandlung thrombo-embolischer Ereignisse, die mit einem pathologischen Thrombozytenverhalten einhergehen, wie z.B. nach Herzklappen- oder Gefäßoperationen, bei tiefen Beinvenenthrombosen, bei thrombotisch-thrombopenischen Purpura, nach Coronarinfarkt, nach Cerebralinfarkt, bei sogn. transient ischaemic attacks, bei Amaurosis fugax und zur Prophylaxe der Arteriosklerose.

Die Einzeldosis der neuen Kombination setzt sich hierbei zweckmäßigerweise aus der jeweils am Menschen gebräuchlichen Einzeldosis des verwendeten PDE-Hemmers und des Thromboxan $A_2$-Antagonisten zusammen. Hierbei kann insbesondere auf Grund der guten Verträglichkeit die Dosis des Thromboxan $A_2$-Antagonisten in einem großen Dosisbereich, beispielsweise zwischen 10 und 1000 mg, variiert werden.

Am Beispiel der vorstehend geprüften Kombination bedeutet dies, daß die Einzeldosis dieser Kombination 50 - 100 mg Substanz A, vorzugsweise jedoch 75 mg Substanz A, plus 10-200 mg Substanz B, vorzugsweise 10 bis 100 mg Substanz B, bei einer 3- bis 4- mal täglichen Applikation enthält.

Zur pharmazeutischen Anwendung läßt sich die neue Kombination, enthaltend 1 bis 500 mg eines PDE-Hemmers, vorzugsweise jedoch 2 bis 75 mg, plus 10 bis 300 mg eines Thromboxan/$A_2$-Antagonisten, vorzugsweise jedoch 10 bis 200 mg, sowie deren physiologisch verträgliche Additionssalze zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Zur Erzielung einer entsprechen Wirkung am Erwachsenen erfolgt die Applikation der vorstehend erwähnten Einzeldosis 2 bis 4 mal täglich, vorzugsweise jedoch 3 bis 4 mal täglich.

Die nachfolgenden Beispiele sollen die Erfindung näher erläutern:

Beispiel 1

Filmtabletten, enthaltend 75 mg PDE-Hemmer + 75 mg Thromboxan $A_2$-Antagonist

Eine Pulvermischung aus

Dipyridamol 25 %
4-[2-(Benzolsulfonamido)-äthyl)]-phenoxy-essigsäure 25 %
Fumarsäure 15 %
Cellulose 20 %
Maisstärke 8 %
Polyvinylpyrrolidon 6 %

wird in einem Mischgerät mit Wasser befeuchtet und durch ein Sieb mit der Maschenweite 1.5 mm granuliert. Nach Trocknung und erneuter Siebung mischt man 1 % Magnesiumstearat zu und stellt 10 mm bikonvexe Tabletten von 300 mg her. Diese Tabletten werden solange mit Hydroxypropylmethylcelluloselack besprüht bis sie 312 mg wiegen.

Beispiel 2

Hartgelatinekapseln, enthaltend 50 mg PDE-Hemmer + 200 mg Thromboxan $A_2$-Antagonist

10 kg Dipyridamol, 20 kg Fumarsäure, 11,5 kg Polyvinylpyrrolidon, 40 kg 3-[4-(2-(p-Toluolsulfonamido]-äthyl]-benzoyl]-propionsäure, 1,5 kg Siliciumdioxid und 0,8 kg Magnesiumstearat mischt man 15 Minuten in einem Kubusmischer. Diese Mischung gibt man über einen Walzenkompaktor, dem ein Trockengranuliergerät mit Siebeinrichtung nachgeschaltet ist. Verwendung findet die Fraktion 0.25-1,0 mm. Die Kapselfüllmaschine wird so eingestellt, daß jede Kapsel der Größe 0 die 50 mg PDE-Hemmer und 200 mg Thromboxan $A_2$-Antagonist entsprechende Menge Granulat enthält.

Beispiel 3

Hartgelatinekapseln enthaltend 250 mg PDE-Hemmer + 100 mg Thromboxan $A_2$-Antagonist

a) Granulat

125 kg Mopidamol, 50 kg Fumarsäure, 13,5 kg Milchzucker werden gemischt und mit einer Lösung aus Wasser/Polyäthylenglykol 6000 befeuchtet. Nach Granulierung durch ein Sieb mit der Maschenweite 1,0 mm und Trocknung bei 45°C mischt man 1,4 kg Stearinsäure zu.

b) Dragée

100 kg 3-[4-(2-Benzolsulfonylaminoäthyl)-benzoyl]propionsäure-Kaliumsalz, 7,5 kg Hydroxypropylmethylcellulose, 2,5 kg Siliciumdioxid und 15 kg Carboxymethylcellulose werden mit Äthanol befeuchtet und durch ein Sieb mit der Maschenweite 1,5 mm granuliert. Nach Trocknung mischt man 1 kg Magnesiumstearat zu und verpreßt das Granulat zu 126 mg schweren bikonvexen Tabletten mit einem Durchmesser von 5,5 mm.
Diese Kerne überzieht man in mehreren Schritten mit einer Dragiersuspension, bestehend aus 5,6 kg Saccharose, 0,5 kg Gummi-arabicum und 3,8 kg Talcum, bis die Tabletten ein Gewicht von 135 mg haben.

c) Abfüllung

Auf einer Spezial-Kapselmaschine füllt man in eine Hartgelatine-Kapsel der Größe 0 long die 250 mg PDE-Hemmer entsprechende Menge Granulat ein und legt das 100 mg Thromboxan $A_2$-Antagonist enthaltende Dragée obenauf.

Beispiel 4

Suspension, enthaltend 100 mg Dipyridamol + 10 mg Thromboxan $A_2$-Antagonist pro 5 g.

Die Suspension hat folgende Zusammensetzung:

(1) Dipyridamol          2,0 %
(2) 3-[4-(2-(4-Fluorbenzolsulfonylamino)-äthyl)-benzoyl]-propionsäure 0,2 %
(3) Sorbit          20,8 %
(4) Cellulose          7,5 %
(5) Natriumcarboxymethylcellulose          2,5 %
(6) Geschmackskorrigentien/Konservierungsstoffe          1,8 %
(7) Wasser          65,2 %
In heißes Wasser wird unter hoher Scherung (3) - (6) eingerührt. Nach Abkühlung werden in die viskose Suspension (1), (2) und (7) eingearbeitet.
5 g dieser Suspension enthalten 100 mg PDE-Hemmer und 10 mg Thromboxan $A_2$-Antagonist.

Beispiel 5

Depot-Form, enthaltend 200 mg Dipyridamol und 50 mg Thromboxan $A_2$-Antagonist.

6

a) Pellet I

Eine Mischung aus

4-[2-(Benzolsulfonamido)-äthyl]-phenoxy-essigsäure        50,0 kg
Lysin        12,5 kg
Hydroxypropylcellulose, hochpolymer        52,5 kg
Triacetin        4,0 kg
Äthylcellulose        2,5 kg
Magnesiumstearat        3,5 kg
wird auf einem Spezialextruder mit Äthanol geknetet und in Form von Spaghetti (Durchmesser 1 mm) extrudiert, die in einem Sphäronizer zu Pellets gerundet werden. Danach trocknet man sie gründlich.

b) Pellet II

300 kg ausgemischte Weinsäure-Starterpellets werden in einem Spezialkessel mit einer Suspension bestehend aus Isopropanol, Dipyridamol und Polyvinylpyrrolidon solange besprüht, bis die entstehenden Wirkstoffpellets ca. 45 % Dipyridamol enthalten.

Diese Pellets besprüht man mit einem Lack, der aus Methacrylsäure/Methylmethacrylat-Copolymer (Handelsnahme Eudragit S) und Hydroxypropylmethylcellulosephthalat (Handelsname HP 55) im Gewichtsverhältnis 85:15 bis 50:50 besteht. Die organischen Lacklösungen enthalten noch Weichmacher und Talkum. Es werden zwei Pelletkomponenten mit 5 und 7 % Hüllenmittel und unterschiedlichem Verhältnis der Lackkomponenten in den genannten Grenzen gesprüht. Die beiden Komponenten werden so gemischt, daß sie nachfolgende in vitro-Freigabe ergeben:
Bedingungen (entsprechend USPXXI, Basket-Methode, 100 Umdrehungen/Min.,
1. Stunde künstlicher Magensaft, pH 1,2, 2. bis 6. Stunde künstlicher Darmsaft (Phosphatbuffer), pH 5.5):

Wirkstoff-Freigabe pro Stunde:

1. Stunde        ca.30 %
2. Stunde        ca. 25 %
3. Stunde        ca. 18 %
4. Stunde        ca. 12 %
nach der 6. Stunde mehr als 90 % Dipyridamol-Freigabe.

c) Abfüllung

Entsprechend dem Wirkstoffgehalt der Pelletkomponenten I und II und der gewünschten Dosierung werden die Pellets miteinander vermischt und auf einer Kapselmaschine in Kapseln der Größe 0 long abgefüllt. Die Kapsel enthält 200 mg PDE-Hemmer und 50 mg Thromboxan $A_2$-Antagonist in verzögert freigebender Form.

Beispiel 6

Ampullen, enthaltend 10 mg PDE-Hemmer + 5 mg Thromboxan $A_2$-Antagonist per 5 ml

Zusammensetzung:

(1) Dipyridamol        10 mg
(2) 4-[4-(2-Benzolsulfonylamino-äthyl)-phenyl]-4-hydroxy-buttersäure        5 mg
(3) Propylenglykol        50 mg
(4) Polyäthylenglykol        5 mg
(5) Äthanol        10 mg
(6) Wasser für Injektionszwecke ad        5 ml
(7) Verd. HCl ad        pH 3
Die Wirkstoffe werden unter Erwärmung in der Lösung aus (3) - (7) gelöst. Nach pH-Kontrolle und Sterilfiltration füllt man in geeignete Ampullen und sterilisiert.

Beispiel 7

Suppositorien, enthaltend 100 mg PDE-Hemmer + 200 mg Thromboxan A$_2$-Antagonist

1 Suppositorium enthält:

    (1) Papaverin        100 mg
    (2) 3-[4-(2-(p-Toluolsulfonylamino)-äthyl)-benzoyl]-propionsäure-methylester       200 mg
    (3) Fumarsäure      200 mg
    Suppositorienmasse     1 500 mg

    In die geschmolzene Masse werden bei 50°C die mikronisierten Komponenten (1) - (3) agglomeratfrei suspendiert. Es werden Suppositorien mit 2 g Gewicht gegossen.

    Selbstverständlich können auch andere PDE-Hemmer und Thromboxan A$_2$-Antagonisten in den in der Beschreibung erwähnten Dosierungen in die vorstehend beschriebenen pharmazeutischen Anwendungsbeispiele eingearbeitet werden.

**Patentansprüche**

1.    Synergistische antithrombotische Arzneimittelkombination, enthaltend einen Phosphodiesterasehemmer und einen Thromboxan A$_2$-Antagonisten neben einem oder mehreren inerten Trägerstoffen.

2.    Arzneimittelkombination gemäß Anspruch 1, dadurch gekennzeichnet, daß diese als PDE-Hemmer einen aus der Pyrimido[5,4-d]pyrimidin-Reihe, aus der Isochinolinreihe, aus der Pyrido[3,2-d]-pyridiminreihe, aus der Indolin- und Carbostyrilreihe und der Benzoxazin-2-on-Reihe enthält.

3.    Arzneimittelkombination gemäß Anspruch 1, dadurch gekennzeichnet, daß diese als PDE-Hemmer einen aus der Pyrimido[5,4-d]pyrimidin-Reihe enthält.

4.    Arzneimittelkombination gemäß den Anspruch 3, dadurch gekennzeichnet, daß diese als PDE-Hemmer Dipyridamol enthält.

5.    Verwendung einer Arzneimittelkombination gemäß den Ansprüchen 1 bis 4 zur Behandlung und Prophylaxe von venösen und arteriellen Thrombosen.

6.    Verwendung einer Kombination bestehend aus einem PDE-Hemmer und einem Thromboxan A$_2$-Antagonisten zur Herstellung eines Arzneimittels, welches zur Behandlung und Prophylaxe von venösen und arteriellen Thrombosen geeignet ist.

7.    Verfahren zur Herstellung eines Arzneimittels gemäß den Ansprüchen 1 bis 4, dadurch gekennzeichnet, daß auf nichtchemischem Wege ein PDE-Hemmer und ein Thromboxan A$_2$-Antagonist in einen oder mehrere inerte übliche Trägerstoffe eingearbeitet wird.

**Claims**

1.    Synergistic antithrombotic pharmaceutical combination containing a phosphodiesterase inhibitor and a thromboxane A$_2$ antagonist together with one or more inert carriers.

2.    Pharmaceutical combination as claimed in claim 1, characterized in that it contains as PDE inhibitor a PDE inhibitor selected from the pyrimido[5,4-d]pyrimidine series, from the isoquinoline series, from the pyrido[3,2-d]pyrimidine series, from the indoline and carbostyril series and the benzoxazin-2-one series.

3.    Pharmaceutical combination as claimed in claim 1, characterised in that it contains as PDE inhibitor one selected from the pyrimido[5,4-d]pyrimidine series.

4.    Pharmaceutical combination as claimed in claim 3, characterised in that it contains dipyridamole as PDE inhibitor.

5.    Use of a pharmaceutical combination as claimed in claims 1 to 4 for the treatment and prophylaxis of venous and arterial thrombosis.

6. Use of a combination consisting of a PDE inhibitor and a thromboxane $A_2$ antagonist for the preparation of a pharmaceutical composition which is suitable for the treatment and prophylaxis or venous and arterial thrombosis.

7. Process for preparing a pharmaceutical combination as claimed in claims 1 to 4, characterised in that a PDE inhibitor and a thromboxane $A_2$ antagonist are incorporated in one or more inert conventional carriers by a non-chemical method.

**Revendications**

1. Combinaison médicamenteuse synergique antithrombotique contenant un inhibiteur de la phosphodiestérase et un antagoniste du thromboxane $A_2$ ainsi qu'un ou plusieurs véhicules inertes.

2. Combinaison médicamenteuse selon la revendication 1, caractérisée en ce qu'elle contient comme inhibiteur de la PDE un inhibiteur de la série de la pyrimido-[5,4-d]pyrimidine, de la série de l'isoquinoléine, de la série de la pyrido[3,2-d]pyridimine, de la série de l'indoléine et du carbostyrile et de la série de la benzoxazinone-2.

3. Combinaison médicamenteuse selon la revendication 1, caractérisée en ce qu'elle contient comme inhibiteur de la PDE un inhibiteur de la série de la pyrimido-[5,4-d]pyrimidine.

4. Combinaison médicamenteuse selon la revendication 3, caractérisée en ce qu'elle contient du dipyridamol comme inhibiteur de la PDE.

5. Utilisation d'une combinaison médicamenteuse selon les revendications 1 à 4 pour le traitement et la prophylaxie des thromboses veineuses et artérielles.

6. Utilisation d'une combinaison consistant en un inhibiteur de la PDE et en un antagoniste du thromboxa-ne $A_2$ pour la préparation d'un médicament qui convient pour le traitement et la prophylaxie des thromboses veineuses et artérielles.

7. Procédé de préparation d'un médicament selon les revendications 1 à 4, caractérisé en ce qu'un inhibiteur de la PDE et un antagoniste du thromboxane $A_2$ sont incorporés par voie non chimique dans un ou plusieurs véhicules inertes habituels.